# EUROPEAN PATENT APPLICATION

(11) **EP 4 765 151 A1**
(43) Date of publication of application: **24.06.2026**
(21) Application number: 24307155.2
(22) Date of filing: 18.12.2024
(51) Int. Cl.: G16H 30/40, G16H 50/70

(54) **METHOD FOR TRAINING A MACHINE LEARNING MODEL TO DETERMINE THE OPTIMAL DOSE OF A CONTRAST AGENT OR A RADIOACTIVE TRACER**

(71) Applicant: Guerbet, 93420 Villepinte (FR)
(72) Inventor: The designation of the inventor has not yet been filed
(74) Representative: Plasseraud IP

(57) **Abstract**

The invention refers to a computer-implemented method for training a machine learning model to determine the optimal dose of a contrast agent or a radioactive tracer, referred to as an agent, to administer to a patient during a medical imaging session, the method comprising:
- obtaining (S1T) a first set of medical images from a first visit imaging session, wherein the first set includes at least one image using a post-agent sequence acquired after administering a first dose of agent,
- obtaining (S2T) a second set of medical image from a second visit imaging session, the second set comprising multiple subsequent sets of medical images from subsequent imaging sessions, wherein each subsequent set included at least one image using a post-agent sequence acquired after administering a second dose, each subsequent imaging session involving the administration of a second dose of the agent, wherein the sum of the second doses administered across the subsequent sessions is equal or similar to the first dose,
- analyzing (S3T) the acquired sets of subsequent medical images from the subsequent imaging sessions to evaluate the optimal dose to be administered to the patient during a medical imaging session,
- training (S4T) the machine learning model, using the first set of medical images, the first dose, and the evaluated optimal dose, to learn to determine an optimal dose, based on the first set of medical images and the first dose.

## Description

### Technical Field

The present invention relates to the field of medical imaging, specifically to methods for determining the optimal dose of a contrast agent or a radioactive tracer to be administered to a patient during a medical imaging session. The invention aims to personalize the agent dose based on patient characteristics and the pathology under investigation, while minimizing potential risks associated with the use of contrast agents or radioactive tracers.

### Background Art

Magnetic Resonance Imaging (MRI) is a non-invasive diagnostic tool that utilizes magnetic fields and radio waves to generate detailed images of the body's internal structures. Contrast agents, often containing gadolinium, are used to improve the visibility of certain tissues, blood vessels, or pathologies. These agents enhance the contrast between normal and abnormal tissue on MRI images, aiding in the detection and characterization of lesions.

Contrast agents, such as gadolinium-based contrast agents (GBCA) are typically administered intravenously prior to image acquisition. The use of these agents is widespread in clinical practice, with dosage levels determined by patient weight. The GBCA injection increases the sensitivity of MRI. One solution to further increase the sensitivity of MRI is to increase the injected quantity of GBCA. However, based on precautionary considerations, clinical guidelines suggest using the minimum dosage that achieves a sufficient contrast enhancement and GBCA usage should therefore be as parsimonious as possible.

Reducing the dosage of contrast agents can adversely affect the quality of the resulting images, which in turn can impact the accuracy of medical diagnoses. For example, administering half the standard dose may result in small brain metastases remaining undetected, whereas they would be clearly visible with a full dose. This presents a challenge in maintaining diagnostic quality while minimizing the GBCA dosage.

Efforts to address this challenge have included the development of image processing methods that employ deep learning technology to enhance the quality of images obtained with lower doses of contrast agents. "Gong E, Pauly JM, Wintermark M, Zaharchuk G. Deep learning enables reduced gadolinium dose for contrast-enhanced brain MRI. J Magn Reson Imaging. 2018 Aug;48(2):330-340. doi: 10.1002/jmri.25970. Epub 2018 Feb 13. PMID: 29437269" presents one such approach, and subsequent publications have introduced a variety of similar methods. However, these techniques, while successful in improving image quality to some extent, have not consistently achieved the level of enhancement necessary to ensure accurate diagnoses across a broad patient population, as indicated by "Ammari S et al., Can Deep Learning Replace Gadolinium in Neuro-Oncology?: A Reader Study. Invest Radiol. 2022 Feb 1;57(2):99-107. doi: 10.1097/RLI.0000000000000811. PMID: 34324463" and "Jayachandran Preetha C et al., Deep-learning-based synthesis of post-contrast T1-weighted MRI for tumour response assessment in neuro-oncology: a multicentre, retrospective cohort study. Lancet Digit Health. 2021 Dec;3(12):e784-e794. doi: 10.1016/S2589-7500(21)00205-3. Epub 2021 Oct 20. PMID: 34688602".

The same challenges and considerations apply to other modalities using contrast agents, such as Computed Tomography (CT), and to the use of radioactive tracers in different imaging modalities, such as Positron Emission Tomography (PET) and Single Photon Emission Computed Tomography (SPECT). Radioactive tracers are substances that emit radiation and are used to highlight metabolic activity and detect abnormalities such as cancer. These tracers are typically administered intravenously and accumulate in specific tissues or organs, allowing for detailed imaging of physiological processes. However, the use of radioactive tracers poses health risks due to radiation exposure, and there is a significant interest in reducing the dosage to minimize these risks. Similar to contrast agents in MRI or in CT imaging, reducing the dosage of radioactive tracers can compromise image quality and diagnostic accuracy. Therefore, there is a need for improved methods to enhance image quality when using lower doses of radioactive tracers, leveraging existing imaging data and advanced image processing techniques to ensure accurate diagnoses while minimizing radiation exposure.

The interest in reducing contrast agent or radioactive tracer is even more acute in the case of returning patients that undergo follow-up imaging sessions to monitor a known disease or chronic condition. Indeed, their compound contrast agent or radioactive tracer intake increases at each new imaging session, by a marginal quantity that should be as small as possible.

There is a need for an improved method of determining the optimal dose of contrast agents or radioactive tracers that overcomes the limitations of current practices, which often rely solely on patient weight. There is also a need to personalize the dose based on individual patient characteristics and the specific pathology being investigated to enhance diagnostic accuracy while minimizing potential risks.

### Summary

To that aim, the present document proposes a computer-implemented method for training a machine learning model to determine the optimal dose of a contrast agent or a radioactive tracer, referred to as an agent, to administer to a patient during a medical imaging session, the method comprising:
- obtaining a first set of medical images from a first visit imaging session, wherein the first set includes at least one image using a post-agent sequence acquired after administering a first dose of agent,
- obtaining a second set of medical image from a second visit imaging session, the second set comprising multiple subsequent sets of medical images from subsequent imaging sessions, wherein each subsequent set included at least one image using a post-agent sequence acquired after administering a second dose, each subsequent imaging session involving the administration of a second dose of the agent, wherein the sum of the second doses administered across the subsequent sessions is equal or similar to the first dose,
- analyzing the acquired sets of subsequent medical images from the subsequent imaging sessions to evaluate the optimal dose to be administered to the patient during a medical imaging session,
- training the machine learning model, using the first set of medical images, the first dose, and the evaluated optimal dose, to learn to determine an optimal dose, based on the first set of medical images and the first dose.

In the context of the present invention, it should be understood that the terms "a," "an," and "at least one" are used interchangeably unless otherwise specified. Specifically, the use of the term "a" or "an" does not limit the invention to a single element but should be interpreted as including the possibility of multiple elements, unless clearly indicated otherwise. Therefore, the term "at least one" also encompasses the situation where only one element is present.

A medical image may be defined as a visual representation of internal body structures or functions acquired using various imaging modalities. These images are generated through the use of hardware and software systems that capture and process data, producing two-dimensional (2D) or three-dimensional (3D) images that aid in the diagnosis, treatment planning, and monitoring of various medical conditions. Medical images typically contain information about tissue density, composition, and function and are interpreted by radiologists, physicians, or other medical professionals with specialized training in image analysis.

A contrast agent is a substance used in medical imaging to enhance the contrast of structures or fluids within the body, thereby improving the visibility of specific areas in the resulting images. In magnetic resonance imaging (MRI), contrast agents often contain gadolinium, which helps to differentiate between normal and abnormal tissues, blood vessels, or pathologies by altering the local magnetic properties and thereby enhancing the image contrast. These agents may typically be administered intravenously or orally before the imaging procedure.

A radioactive tracer is a substance that contains a radioactive isotope and is used in medical imaging to highlight metabolic activity and detect abnormalities such as cancer. These tracers emit radiation and are typically administered intravenously or orally, accumulating in specific tissues or organs, allowing for detailed imaging of physiological processes thro ugh modalities like Positron Emission Tomography (PET) and Single Photon Emission Computed Tomography (SPECT).

An optimal dose refers to the specific amount of a contrast agent or radioactive tracer that achieves the best balance between image quality and safety during a medical imaging session. It is determined based on individual patient characteristics and the pathology under investigation, aiming to enhance diagnostic accuracy while minimizing potential risks associated with the use of these agents. In other words, it may correspond to the minimum dose that allows a correct diagnosis.

A first visit imagining session refers to a medical imaging conducted prior to a follow-up imaging session. The images from this session serve as a reference point for comparison with images from subsequent follow-up sessions to monitor changes or assess the effectiveness of treatments.

A subsequent imaging session refers to a subsequent medical imaging session conducted after an initial first visit imaging session. This session typically occurs within a specified time frame, such as 2 to 6 months after the first visit imaging session and follows a similar imaging protocol. The purpose of the subsequent imaging session is to monitor changes, assess the effectiveness of treatments, or further investigate findings from the first visit session.

A post-agent sequence refers to a series of medical images acquired after the administration of the agent. These sequences are used to enhance the visibility of specific tissues, blood vessels, or pathologies by increasing the contrast between different structures in the resulting images.

Training a machine learning model refers to the process of teaching a machine learning algorithm to make accurate predictions or decisions based on data. This involves feeding the model a set of data (training data) and allowing it to learn the relationships and patterns within the data through iterative optimization techniques. The goal is to adjust the model's parameters to minimize errors and improve its performance on new, unseen data.

By obtaining a first set of medical images from a first visit imaging session, which includes at least one image using a post-agent sequence acquired after administering a first dose of agent, the method ensures that the machine learning model has a baseline reference of the ability of the patient's internal structures to enhance after contrast agent injection. This baseline may be important for accurate estimation of the patient-specific optimal dose in subsequent imaging sessions.

Obtaining multiple subsequent sets of medical images from subsequent imaging sessions, where each set includes at least one image using a post-agent sequence acquired after administering a second dose, allows the method to gather comprehensive data over the effect of varying doses of the agent on image quality.

Analyzing the acquired sets of subsequent medical images to evaluate the optimal dose to be administered to the patient during a medical imaging session leverages the acquired data to personalize the agent dose. This personalized approach aims to balance image quality and patient safety, reducing the risks associated with high doses of contrast agents or radioactive tracers while maintaining diagnostic accuracy.

Training the machine learning model using the first set of medical images, the first dose, and the evaluated optimal dose enables the model to learn from real patient data, improving its ability to predict the optimal dose for future patients. This training process enhances the model's accuracy and reliability in determining the appropriate dose, leading to better diagnostic outcomes and more efficient use of contrast agents or radioactive tracers.

The image may be a 3D image. The 3D image may be composed of a stack of slices, each slice forming a 2D image. Each slice represents a thin cross-section of the organ or the body of the patient, and when these slices are arranged in order, they form a 3D reconstruction of the organ or the body.

A medical image may take many different forms, depending on the imaging modality used and the type of medical condition being investigated. According to the present document, the image may be issued from different modalities such as, but not limited to the following illustrative examples:
- Magnetic Resonance Imaging (MRI): Contrast agents, often containing gadolinium, are used to improve the visibility of tissues, blood vessels, and lesions. They enhance the contrast between normal and abnormal tissue on MRI images;
- Computed Tomography (CT) Scans: Iodine-based contrast agents may for example be used to highlight blood vessels, organs, and other structures, providing detailed information about the internal anatomy;
- X-ray Radiography: Contrast agents, such as barium or iodine-based compounds, may be used in X-ray radiography to visualize the gastrointestinal tract, blood vessels, and other structures;
- Ultrasound (Sonography): Microbubble contrast agents may be used in ultrasound imaging to enhance the visualization of blood flow and tissue perfusion, particularly in cardiac and liver imaging;
- Positron Emission Tomography (PET): Radioactive tracers, which act as contrast agents, may be used in PET scans to highlight metabolic activity and detect abnormalities such as cancer; and
- Fluoroscopy: Iodine-based contrast agents may be used in fluoroscopy to visualize real-time moving images of internal structures, such as during angiography or gastrointestinal studies.

The administration of the agent, for example, intravenously, is not part of the method described herein. The method focuses solely on obtaining (i.e. retrieving) medical images and processing these images, using for example, machine learning techniques.

Depending on the image modality, examples of the contrast agent may include, but are not limited to the following:
- Radiography and Computed Tomography (CT scan): Iodinated contrast agents, such as lohexol ethiodized oil (Lipiodol), (Omnipaque), lopamidol (lopamiron), lopromide (Ultravist), lodixanol (Visipaque), loversol (Optiray), lomeprol (lomeron), lobitridol (Xenetix), loxaglate (Hexabrix), lotrolan (Isovist), and loxilan (Oxilan). Barium sulfate, including Micropaque, Baritop, and E-Z-HD;
- Magnetic Resonance Imaging (MRI): Gadolinium-based chelates, such as Gadopiclenol (Elucirem, VueWay), Gadopentetate dimeglumine (Magnevist), Gadoterate meglumine (Dotarem), Gadodiamide (Omniscan), Gadobutrol (Gadovist), BAY 1747846 (Gadoquatrane), Gadoteridol (ProHance), Gadobenate dimeglumine (MultiHance), Gadoxetate disodium (Primovist), and Gadofosveset trisodium (Ablavar). Iron-based agents, such as Ferumoxtran-10; and
- Ultrasound: Ultrasound contrast agents, such as Sulfur hexafluoride (SonoVue), Perflutren (Definity), and Perflubutane (Sonazoid), perflutren protein-type A microspheres (Optison), sulfur hexafluoride lipid-type A microspheres (Lumason), perflutren (Definity).

Depending on the imaging modality the radioactive agent may be a radiopharmaceutical type such as, but not limited to, the following:
- Positron Emission Tomography (PET): Fluorine-18 (^18F), including Fluorodeoxyglucose (^18F-FDG), Sodium Fluoride (^18F-NaF), and Fluorocholine (^18F-FCH). Carbon-11 (^11C), including Methionine (^11C-MET) and Acetate (^11C-AC). Gallium-68 (^68Ga), such as Dotatate (^68Ga-DOTATATE) and PSMA-11 (^68Ga-PSMA-11), Fluorine-18 (^18F), including Fluorodeoxyglucose (^18F-FDG), Sodium Fluoride (^18F-NaF), and Fluorocholine (^18F-FCH), and Pylarify (^18F-DCFPyl), and Posluma (^18F-RhPSMA-7.3) and RADELUMIN (^18F PSMA-1007) and PYLCLARI (^18F piflufolastat);
- Single Photon Emission Computed Tomography (SPECT): Technetium-99m (^99mTc), including Pertechnetate (^99mTcO4^-), Medronate (^99mTc-MDP), Sestamibi (^99mTc-MIBI), and Macroaggregated Albumin (^99mTc-MAA). Iodine-123 (^123l), such as lobenguane (^1231-MIBG) and Sodium Iodide (^123l-Nal). Thallium-201 (^201Tl), in the form of Thallium Chloride (^201TlCl) ; and
- Scintigraphy: Indium-111 (^111In), including Octreotide (^111In-pentetreotide) and Labeled Leukocytes (^111In). Gallium-67 (^67Ga), such as Gallium Citrate (^67Ga).

The time intervals between the subsequent images may be adjusted based on the type of agent used. For example, gadolinium-based contrast agents (GBCA) may require different time intervals compared to iodinated contrast agents or radioactive tracers. A person skilled in the art, such as an experienced professional in the field of medical imaging, will know how to appropriately adjust these intervals for each type of agent to optimize the quality of the obtained images and ensure an accurate assessment of the effect of the administered agent. This adaptation may ensure that the captured images accurately reflect the characteristics of the internal tissues and structures, thereby enabling more precise training and inference of the machine learning model.

The sum of the second doses administered across the subsequent sessions is comprised between 0.5 and 1.5 times the first dose, preferably between 0.8 and 1.2 times the first dose, more preferably equal to the first dose.

The last subsequent set of medical images may be acquired after a duration of maximum 30 minutes after administering the second dose of agent of the first subsequent medical imaging session.

The duration between the administration of the agent and the acquisition of the last subsequent set of medical images may be longer depending on the type of agent used.

By specifying that the last subsequent set of medical images is acquired after a maximum duration following the administration of the second dose of the agent, the method ensures that the timing of image acquisition is optimized to capture the most relevant and high-quality images. This timing consideration may be important for accurately assessing the effect of the administered agent and for training the machine learning model with consistent and reliable data. It helps in maintaining the integrity of the imaging protocol and ensures that the images used for training reflect the optimal contrast enhancement, leading to better model performance and more accurate dose determination.

The machine learning model may be a CNN.

A Convolutional Neural Network (CNN) is a type of deep learning algorithm specifically designed for processing structured grid data, such as images. It consists of multiple layers, which are mostly convolutional layers that apply filters to the input data to detect features, or pooling layers that reduce the dimensionality of the data. CNNs are particularly effective for image recognition and processing tasks due to their ability to automatically and adaptively learn spatial hierarchies of features from input images.

The machine learning model may be a U-Net.

A U-Net is a convolutional neural network (CNN) architecture introduced by the article "Olaf Ronneberger et al., U-Net: Convolutional Networks for Biomedical Image Segmentation, arXiv: 1505.04597".

The U-Net architecture is characterized by its U-shaped architecture, which consists of an encoder (contracting or downsampling path) and a decoder (expansive path). A key innovation of the U-Net architecture is the use of skip connections, also known as residual connections. These connections allow the decoder to combine high-level semantic information with detailed spatial information from the encoder.

The machine learning model may comprise an input layer that accepts volumetric data with 7 channels. The machine learning model may use 3x3x3 convolutions throughout its layers, for example with PReLU activations in the intermediate layers, and 2x2x2 striding for downsampling. Transposed convolutions may be used for upsampling path (decoder), ensuring symmetry between the encoder and decoder. Such configuration effectively processes and segments 3D medical images with 8 input channels, capturing complex volumetric features.

The first dose, consisting as a single number rather than a full volumetric data, may be inputted to the machine learning model as an 8^{th} channel of the input layer, by repeating the dose number on all dimensions. A similar approach may also be used to aggregate the value of the first dose into intermediate and/or final feature layers, resolving the dimensional mismatch either by simply replicating the same value as many times as necessary, or by relying on additional learnable parameters as proposed in, for instance: Pinetz, Thomas, et al. "Faithful Synthesis of Low-Dose Contrast-Enhanced Brain MRI Scans Using Noise-Preserving Conditional GANs." International Conference on Medical Image Computing and Computer-Assisted Intervention. Cham: Springer Nature Switzerland, 2023.

The first set of medical images may also comprise at least one image using a pre-agent sequence.

The second set of medical images may also comprise at least one image using a pre-agent sequence. The pre-agent image may be common to all the subsequent images.

A pre-agent sequence refers to a series of medical imaging scans that are acquired before the administration of a contrast agent or a radioactive tracer. These sequences provide baseline images of the tissues and structures without the enhanced visibility that said agent provide, allowing for comparison with post-agent images to assess the effect of the agent and identify abnormalities.

By including at least one image using a pre-agent sequence, the method provides the machine learning model with images that represent the tissues and structures without the enhanced visibility provided by the agent. This helps the model to better understand the natural state of the tissues and structures, which is useful for accurately predicting the optimal dose for a given patient.

Acquiring the images may be performed by Magnetic Resonance Imaging, in which the first set and each of the subsequent sets of medical images may comprise a T1, a T2-Flair and/or an ADC pre-agent sequence image.

T1 refers to a specific type of magnetic resonance imaging (MRI) sequence used to produce images based on the longitudinal relaxation time of tissues. T1-weighted images are particularly useful for visualizing anatomical structures and detecting abnormalities, as they provide high contrast between different types of tissues, such as gray matter, white matter, and cerebrospinal fluid in the case of central nervous system imaging.

T2-Flair (Fluid-Attenuated Inversion Recovery) is a specific type of magnetic resonance imaging (MRI) sequence used to produce images with high contrast between different types of tissues. This sequence is particularly effective in suppressing the signal from cerebrospinal fluid (CSF), making it useful for detecting lesions in the brain, such as those associated with multiple sclerosis, tumors, and other pathologies. By attenuating the fluid signal, T2-FLAIR enhances the visibility of abnormalities that may be obscured in standard T2-weighted images.

An ADC (Apparent Diffusion Coefficient) is a quantitative measurement derived from Diffusion Weighted Imaging (DWI) in magnetic resonance imaging (MRI). It reflects the magnitude of water diffusion within tissue and is used to assess tissue cellularity and the integrity of cellular membranes. ADC maps are particularly useful in identifying and characterizing lesions, such as tumors or areas of ischemia, by highlighting differences in water diffusion between normal and abnormal tissues.

Acquiring the images may be performed by Computed Tomography, the first set and each of the subsequent sets of medical images comprise may comprise an unenhanced scan, an arterial phase, a portal venous phase, and/or a delayed phase.

An unenhanced scan refers to a medical imaging scan performed without the use of contrast agents or radioactive tracers. In the context of Computed Tomography (CT), it involves capturing images of the body's internal structures in their natural state, without any artificial enhancement to improve visibility of specific tissues or abnormalities. This type of scan serves as a baseline for comparison with enhanced scans, which are taken after the administration of contrast agents to highlight certain areas of interest.

The arterial phase refers to a specific time period during a contrast-enhanced imaging procedure, such as Computed Tomography (CT), when the contrast agent is predominantly present in the arteries. This phase typically occurs shortly after the injection of the contrast agent and is used to capture images that highlight arterial structures, providing detailed visualization of blood flow and vascular anatomy.

The portal venous phase is a specific time period during a contrast-enhanced imaging procedure, such as Computed Tomography (CT), where the contrast agent is predominantly present in the portal vein and the liver. This phase typically occurs approximately 60-70 seconds after the injection of the contrast agent and is used to capture images that highlight the portal venous system and liver parenchyma, providing detailed visualization of liver lesions and other abdominal structures.

The delayed phase refers to a specific time period during a contrast-enhanced imaging procedure, such as Computed Tomography (CT), when the contrast agent has diffused into the extracellular space and is predominantly present in tissues rather than blood vessels. This phase typically occurs several minutes after the injection of the contrast agent and is used to capture images that highlight tissue characteristics, providing detailed visualization of lesions, tumors, and other abnormalities.

The present document also proposes a computer-implemented method for determining the optimal dose of a contrast agent or a radioactive tracer, referred to as an agent, to administer to a patient during a medical imaging session using a machine learning model, the method comprising:
- obtaining a first set of medical images from a first visit imaging session, wherein the first set includes at least one image using a post-agent sequence acquired after administering a first dose of agent,
- determining an optimal dose of said agent to administer to said patient using said trained machine learning model, based on the first set of medical images, a value representing the amount of the first dose, and optionally the at least one image using a pre-agent sequence of the second set of medical images, as input of the model.

The described method illustrates the inference phase. The inference phase refers to the stage in the machine learning process where a trained model is used to make predictions or generate outputs based on new, unseen data. In the context of the patent application, it involves using the trained machine learning model to determine an optimal dose of the agent to administerto the patient. This phase contrasts with the training phase, where the model learns from a dataset to optimize its parameters.

The contrast agent may be gadoterate meglumine. In that case, the first dose of the contrast agent may be 0.1 mmol/kg. The optimal dose of contrast agent may be 0.05 mmol/kg.

Alternatively, in one example, the contrast agent may be gadopiclenol, in which the first dose of the contrast agent may be 0.05 mmol/kg. The optimal dose of contrast agent may be 0.025 mmol/kg.

The machine learning model may be trained according to the above described method.

The present document also proposes computer software comprising instructions to implement at least a part of the above-described methods, when the software is executed by a processor.

The present document also proposes a computer device comprising:
- an input interface to receive medical images,
- a memory for storing at least instructions of the computer program,
- a processor accessing the memory for reading the stored instructions and executing at least one of the above-described methods,
- an output interface to provide a trained machine learning model and/or an optimal dose of the agent to administer to the patient (i.e. a numerical value representing said dose).

The present document also proposes a computer-readable non-transient recording medium on which the software is registered to implement at least one of the above-mentioned methods, when the software is executed by a processor.

### Brief Description of Drawings

Other features, details and advantages will be shown in the following detailed description and on the figures, on which:
- Figure 1 schematically shows an example of a computer device according to the present document,
- Figure 2 illustrates a flow-chart of a generic training method according to the present document,
- Figure 3 illustrates a flow-chart of an embodiment of the training method,
- Figure 4 illustrates a flow-chart of a generic inference method according to the present document,
- Figure 5 illustrates a flow-chart of an embodiment of the inference method.

### Description of Embodiments

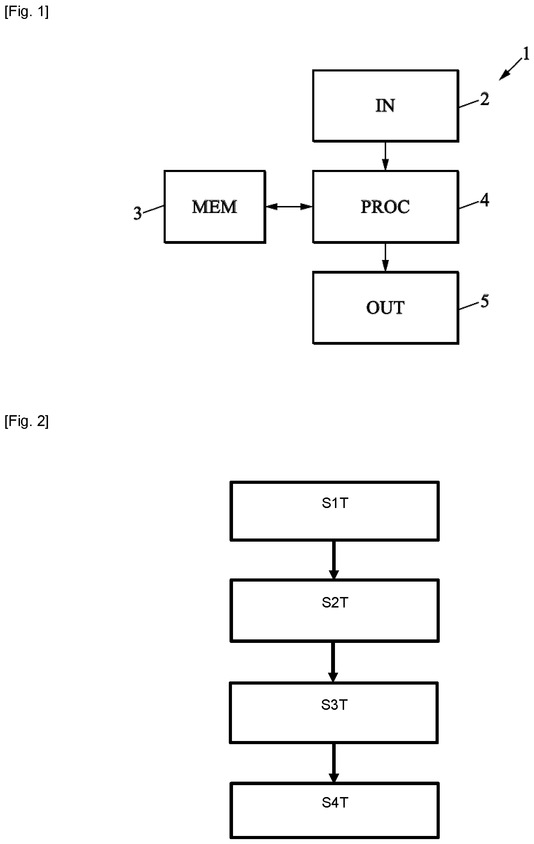

**Figure 1** schematically shows an example of a computer device 1 according to the invention. Said computer device 1 comprises:
- an input interface 2 to receive medical images,
- a memory 3 for storing at least instructions of a computer program,
- a processor 4 accessing memory 3 for reading the aforesaid instructions and executing the training and/or the inference method according to the present document,
- an output interface 5 to provide a trained machine learning model and/or an optimal dose of said agent to administer to said patient.

The input interface 2 serves as the entry point for data into computer device 1. The input interface 2 may support various data formats and communication protocols to accommodate different types of input.

The memory 3 stores data and instructions that the processor 4 may access during operation. The memory 3 may include both volatile and non-volatile memory components, such as RAM, ROM, flash memory, or other types of storage media. The memory 3 may also store the machine learning model and training data.

The processor 4 executes instructions stored in the memory 3 and performs data processing tasks. The processor 4 may include one or more processing units and may be capable of executing a variety of computational tasks, including machine learning algorithms for image enhancement. Processor 4 may also control the flow of data between the input interface 2, memory 3, and output interface 5.

The output interface 5 provides the results of the data processing performed by processor 4. The output interface 5 may deliver the processed data to external devices, systems, or networks. The output may include, but is not limited to, a trained machine learning model and/or an optimal dose of said agent to administer to said patient (i.e. a numerical value representing said dose).

**Figure 2** is a flow chart illustrating the computer-implemented method for training a machine learning model to determine the optimal dose of a contrast agent or a radioactive tracer, referred to as an agent, to administer to a patient during a medical imaging session, the method comprising:
- obtaining (S1T) a first set of medical images from a first visit imaging session, wherein the first set includes at least one image using a post-agent sequence acquired after administering a first dose of agent,
- obtaining (S2T) multiple subsequent sets of medical images from subsequent imaging sessions, wherein each subsequent set included at least one image using a post-agent sequence acquired after administering a second dose, each subsequent imaging session involving the administration of a second dose of the agent, wherein the sum of the second doses administered across the subsequent sessions is equal or similar to the first dose,
- analyzing (S3T) the acquired sets of subsequent medical images from the subsequent imaging sessions to evaluate the optimal dose to be administered to the patient during a medical imaging session,
- training (S4T) the machine learning model, using the first set of medical images and the evaluated optimal dose, to learn to determine an optimal dose, based on the first set of medical images.

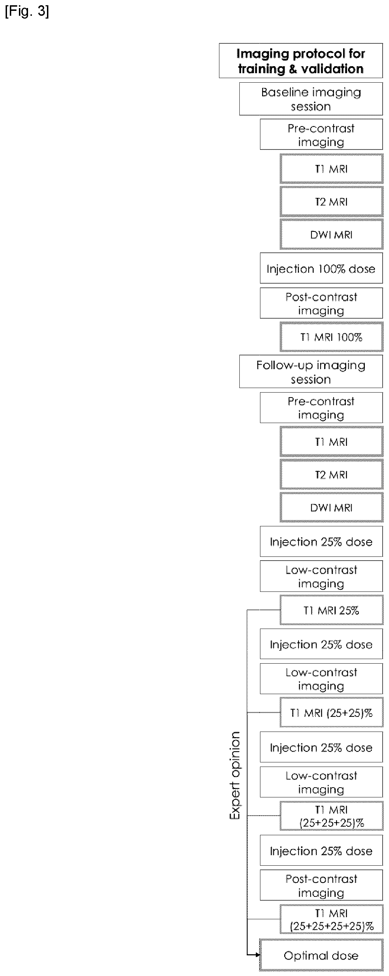

**Figure 3** is a flow chart illustrating a specific embodiment of a part of the training phase, where, for the first visit imaging sessions, pre-contrast MRI sequences are acquired from multiple patients, (example: T1, T2-Flair, and Diffusion Weighted Imaging (DWI) with generated ADC maps). A post-contrast T1c sequence is then acquired, after a first dose of contrast agent (example: 0.1mmol/kg of gadoterate meglumine) is injected intravenously. The images acquired during such first visit imaging sessions constitute the above-mentioned first set of medical images.

The follow-up imaging session is conducted between 2 to 6 months after the first visit imaging session. A similar protocol is followed, but the administration of Gadolinium-Based Contrast Agent is split into four successive injections at 0.025mmol/kg for each injection (the second dose), with an additional "low-dose" gradient echo T1c-low sequence acquired in between. The images acquired during such follow-up imaging session constitute respectively the above-mentioned subsequent sets of medical images.

The the subsequent sets of medical images are analyzed to determine the optimal dose for each patient. A corresponding machine learning model is then trained based on the first set of medical images of patients, the pre-agent images from the follow-up imaging session, the first and optimal doses. The number of patients is for example comprised between 200 and 1000.

The model is a U-Net with an architecture that begins with an input layer designed to accept volumetric data with 8 channels, corresponding to different MRI modalities and the first dose. The network features a symmetric encoder-decoder structure, optimized to capture spatial hierarchies and contextual information effectively.

The downsampling path (encoder) consists of a series of convolutional blocks:
- The first block processes the input using two convolution layers, each with 32 filters of size 3x3x3, stride 1, and padding 1, followed by PReLU activations. This is followed by a 2x2x2 convolution layer with stride 2 for downsampling.
- The second block uses 64 filters in each convolution layer, maintaining the same kernel size and activation functions, and is again followed by stride 2 convolution for downsampling.
- The third block has 128 filters per convolution layer, while the fourth block has 256 filters per layer, both continuing the pattern of 3x3x3 convolutions, PReLU activations, and stride 2 convolution for downsampling.

At the network's bottleneck, the architecture employs two convolution layers with 512 filters each, 3x3x3 kernels, stride 1, and padding 1, followed by PReLU activations.

The upsampling path (decoder) mirrors the encoder but replaces stride 2 convolution with transposed convolutions (deconvolutions) for upsampling:
- The first block in the decoder upsamples using a transposed convolution with 256 filters, a 2x2x2 kernel, and stride 2, followed by two 3x3x3 convolution layers with 256 filters each and PReLU activations.
- The second block uses 128 filters, the third uses 64 filters, and the fourth uses 32 filters per convolution layer, each block preceded by a transposed convolution for upsampling.

The final output layer consists of a 1x1x1 convolution with a single filter.

In summary, the network employs 3x3x3 convolutions throughout its layers, with PReLU activations in the intermediate layers and 2x2x2 stride 2 convolution for downsampling. Transposed convolutions are used for upsampling, ensuring symmetry between the encoder and decoder. This configuration effectively processes and segments 3D medical images with 8 input channels, capturing complex volumetric features.

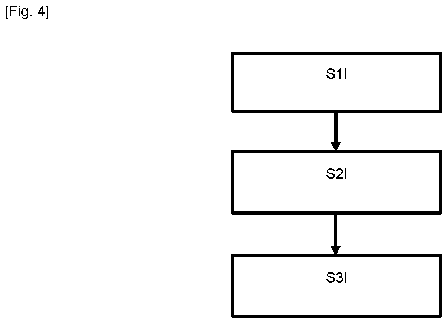

Other model architectures can be used without altering the functionality of the process. **Figure 4** is a flow chart illustrating the computer-implemented method for determining the optimal dose of a contrast agent or a radioactive tracer, referred to as an agent, to administer to a patient during a medical imaging session using a machine learning model, the method comprising:
- obtaining (S1I) a first set of medical images from a first visit imaging session, wherein the first set includes at least one image using a post-agent sequence acquired after administering a first dose of agent,
- optionally, obtaining (S2I) a second set of medical images from a second visit imaging session, wherein the second set includes at least one image using a pre-agent sequence,
- determining (S3I) an optimal dose of the agent to administer to the patient using the trained machine learning model, based on the first set of medical images, a value representing the amount of the first dose, and optionally the at least one image using a pre-agent sequence of the second set of medical image, as input of the model.

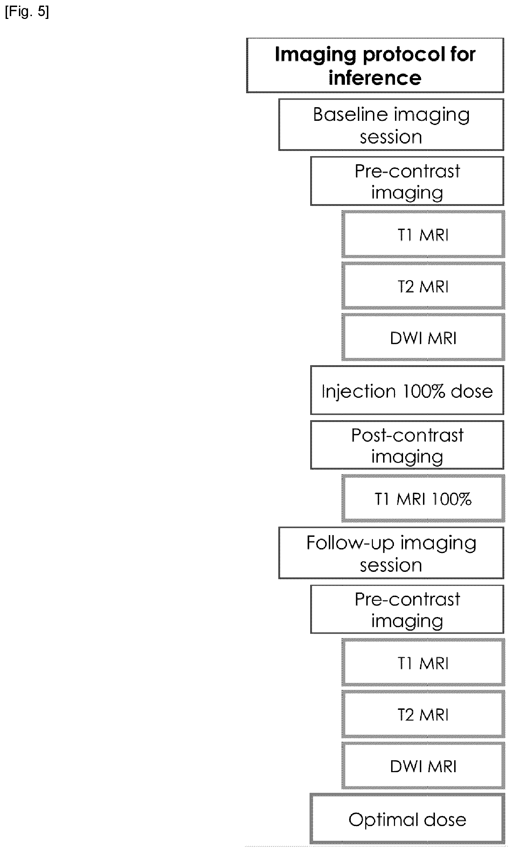

**Figure 5** is a flow chart illustrating a specific embodiment of the inference phase, where pre-contrast MRI sequences are acquired from a new patient during a first visit imaging session, (example: T1, T2-Flair, and Diffusion Weighted Imaging (DWI) with generated ADC maps). A post-contrast T1c sequence is then acquired, after a first dose of contrast agent (example: 0.1mmol/kg of gadoterate meglumine) is injected intravenously. The images acquired during such first visit imaging sessions constitute the above-mentioned first set of medical images of the patient.

At the follow-up or second visit imaging session, the machine learning model predicts the optimal dose that needs to be injected into the patient. At least one image using a pre-agent sequence may be acquired during this follow-up imaging session, (example: T1, T2-Flair, and Diffusion Weighted Imaging (DWI) with generated ADC maps).

## Claims

1. A computer-implemented method for training a machine learning model to determine the optimal dose of a contrast agent or a radioactive tracer, referred to as an agent, to administer to a patient during a medical imaging session, the method comprising:
- obtaining (S1T) a first set of medical images from a first visit imaging session, wherein the first set includes at least one image using a post-agent sequence acquired after administering a first dose of agent,
- obtaining (S2T) a second set of medical image from a second visit imaging session, the second set comprising multiple subsequent sets of medical images from subsequent imaging sessions, wherein each subsequent set included at least one image using a post-agent sequence acquired after administering a second dose, each subsequent imaging session involving the administration of a second dose of the agent, wherein the sum of the second doses administered across the subsequent sessions is equal or similar to the first dose,
- analyzing (S3T) the acquired sets of subsequent medical images from the subsequent imaging sessions to evaluate the optimal dose to be administered to the patient during a medical imaging session,
- training (S4T) the machine learning model, using the first set of medical images, the first dose, and the evaluated optimal dose, to learn to determine an optimal dose, based on the first set of medical images and the first dose.

2. Computer-implemented method according to the preceding claim, wherein the sum of the second doses administered across the subsequent sessions is comprised between 0.5 and 1.5 times the first dose, preferably between 0.8 and 1.2 times the first dose, more preferably equal to the first dose.

3. Computer-implemented method according to any of the preceding claims, wherein the last subsequent set of medical images is acquired after a duration of maximum 30 minutes after administering the second dose of agent of the first subsequent medical imaging session.

4. Computer-implemented method according to any of the preceding claims, wherein the machine learning model is a CNN.

5. Computer-implemented method according to the preceding claims, wherein the machine learning model is a U-Net.

6. Computer-implemented method according to any of the preceding claims, wherein the first set of medical images also comprises at least one image using a pre-agent sequence .

7. Computer-implemented method according to any of the preceding claims, wherein the second set of medical images also comprises at least one image using a pre-agent sequence.

8. Computer-implemented method according to claim 6, wherein acquiring the images is performed by Magnetic Resonance Imaging, the first set and each of the subsequent sets of medical images comprises a T1, a T2-Flair and/or an ADC pre-agent sequence image.

9. Computer-implemented method according to any of the preceding claims, wherein acquiring the images is performed by Magnetic Resonance Imaging, the first set and each of the subsequent sets of medical images comprise a T1 post-agent sequence image.

10. Computer-implemented method according to any of the preceding claims, wherein acquiring the images is performed by Computed Tomography, the first set and each of the subsequent sets of medical images may comprise an unenhanced scan, an arterial phase, a portal venous phase, and/or a delayed phase.

11. A computer-implemented method for determining the optimal dose of a contrast agent or a radioactive tracer, referred to as an agent, to administer to a patient during a medical imaging session using a machine learning model, the method comprising:
- obtaining (S1I) a first set of medical images from a first visit imaging session, wherein the first set includes at least one image using a post-agent sequence acquired after administering a first dose of agent,
- optionally, obtaining (S2I) a second set of medical images from a second visit imaging session, wherein the second set includes at least one image using a pre-agent sequence,
- determining (S3I) an optimal dose of the agent to administer to the patient using the trained machine learning model, based on the first set of medical images, a value representing the amount of the first dose, and optionally the at least one image using a pre-agent sequence of the second set of medical image, as input of the model.

12. Computer-implemented method according to the preceding claim, wherein the machine learning model is trained according to any of the claims 1 to 7.

13. A computer device (1) comprising:
- an input interface (2) to receive medical images,
- a memory (3) for storing at least instructions of a computer program according to the preceding claim,
- a processor (4) accessing memory for reading the stored instructions and executing then the method according to any of the claims 1 to 10 and /or the method according to any of the claims 11 to 14,
- an output interface (5) to provide a trained machine learning model and/or an optimal dose of the agent to administer to the patient.
